Europäisches Patentamt

(19) European Patent Office

Office européen des brevets

(11) Numéro de publication: **0 273 782**
A1

## (12) DEMANDE DE BREVET EUROPEEN

(21) Numéro de dépôt: 87402336.9

(22) Date de dépôt: 19.10.87

(51) Int. Cl.⁴: **C 12 N 15/00**
C 12 N 7/00, C 12 Q 1/70

(30) Priorité: 31.10.86 FR 8615234

(43) Date de publication de la demande:
06.07.88 Bulletin 88/27

(84) Etats contractants désignés:
AT BE CH DE ES FR GB GR IT LI LU NL SE

(71) Demandeur: INSTITUT PASTEUR
25-28, rue du Docteur Roux
F-75724 Paris Cédex 15 (FR)

INSTITUT NATIONAL DE LA SANTE ET DE LA
RECHERCHE MEDICALE (INSERM)
101, rue de Tolbiac
F-75654 Paris Cédex 13 (FR)

(72) Inventeur: Nicolas,Jean-Francois
39,Chemin de la Source
78590 Noisy Le Roi (FR)

Bonnerot, Claire
5bis,rue de Solferino
75007 Paris (FR)

Jacob, Francois
17,rue Conde
75006 Paris (FR)

Rubenstein, John L.
35 rue Lecourbe
75015 Paris (FR)

Sanes Joshua R.
45, rue Mathurin Regnier
75015 Paris (FR)

(74) Mandataire: Gutmann, Ernest et al
S.C. Ernest Gutmann - Yves Plasseraud 67, boulevard
Haussmann
F-75008 Paris (FR)

Le titre de l'invention a été modifié (Directives relatives à l'examen pratiqué à l'OEB, A-III, 7.3)

(54) **Retrovirus recombinant défectif.**

(57) L'invention concerne des moyens pour l'étude des interactions d'un rétrovirus déterminé avec des lignées cellulaires susceptibles d-être infectées par ce rétrovirus. Il met en jeu un rétrovirus recombinant défectif dérivé du rétrovirus en question, contenant les parties agissant en "cis" de ce rétrovirus qui assure normalement son empaquetage, sa réverse-transcription en une molécule ADN et son intégration dans le génome de la cellule-hôte.

Ce rétrovirus recombinant est caractérisé par la substitution partielle ou totale des séquences codant pour ses protéines virales par une séquence génique codant elle-même pour une protéine agissant en tant que marqueur des cellules infectées. La détection du marqueur dans les cellules dont la sensibilité à l'infection est à étudier témoigne de leur capacité à être infectées par le rétrovirus sauvage. Les cellules infectées par le rétrovirus recombinant défectif peuvent, à leur tour, être utilisées comme réactif pour le détection dans du rétrovirus sauvage dans un échantillon biologique extérieur. La présence du rétrovirus sauvage se manifeste par la complémentation en trans du provirus qui est alors produit par ces cellules et qui peut alors être détecté.

EP 0 273 782 A1

**Description**

RETROVIRUS RECOMBINANT DEFECTIF ET CELLULES LE CONTENANT, LEUR PROCEDE DE FABRICATION ET LEURS APPLICATIONS, NOTAMMENT POUR L'ETUDE DE L'INTERACTION DES RETROVIRUS SAUVAGES AVEC DES LIGNEES CELLULAIRES INFECTABLES PAR CELUI-CI OU POUR LA DETECTION IN VITRO D'UNE INFECTION RETROVIRALE

L'invention concerne des moyens (produits et procédés) applicables à l'étude des interactions d'un rétrovirus déterminé avec des lignées cellulaires susceptibles d'être infectées par des virus et, le cas échéant, de l'influence d'agents extérieurs sur cette interaction, ou encore à la détection d'une infection rétrovirale déjà installée au sein d'échantillons de cellules.

Une des variantes d'application de l'invention concerne la détection de la capacité d'un rétrovirus déterminé à infecter une lignée cellulaire donnée, ou l'identification de ceux des clones cellulaires qui, au sein d'une culture cellulaire, sont infectables par ce rétrovirus.

Une autre variante d'application de l'invention vise à permettre l'étude de l'influence d'agents extérieurs sur la capacité d'infection des lignées cellulaires par ce rétrovirus, par exemple de principes actifs de médicaments visant à inhiber le caractère infectieux in vivo de ce rétrovirus.

Une autre variante d'application des moyens selon l'invention se rapporte à la détection de l'existence ou non d'une infection rétrovirale chez un hôte (humain ou animal) sensible à ladite infection rétrovirale et de l'évolution de cette infection, le cas échéant en présence de principes actifs de médicaments.

On conçoit les difficultés que l'on peut rencontrer dans ces types d'étude, lorsque l'on utilise l'agent infectieux lui-même si l'on fait abstraction des risques que peut entraîner sa manipulation pour l'expérimentateur. Certes la capacité d'un rétrovirus à infecter une culture cellulaire déterminée peut être appréciée par la propagation du virus d'une culture à l'autre. Mais ce premier résultat ne permet pas, dans bien des cas, l'identification au sein de la culture cellulaire, de ceux des clones cellulaires qui sont effectivement infectés et qui sont le support direct de la réplication virale. Enfin l'influence éventuelle d'agents extérieurs, tels que des principes actifs de médicaments possibles, sur le degré d'infectiosité où la réplication virale est extrêmement difficile à observer dans des systèmes d'essais mettant en jeu l'agent infectieux lui-même, à supposer que les premières difficultés mentionnées plus haut aient même été surmontées.

L'invention a pour but de remédier au moins en partie à l'ensemble de ces difficultés, notamment de fournir des moyens d'étude qui, substitués à l'agent rétroviral infectieux, permettent néanmoins l'étude du comportement de ce dernier vis-à-vis des hôtes cellulaires qu'il est susceptible d'infecter.

L'invention a aussi pour but de fournir des moyens de ce type qui, en outre, assurent un marquage de ceux des clones cellulaires qui, au sein de la culture étudiée, sont infectables par le rétrovirus considéré.

Elle a encore pour but de fournir des moyens permettant également de détecter, in vitro, l'existence ou non d'une infection rétrovirale, voire même à l'état seulement latent, chez un hôte sensible au rétrovirus correspondant.

L'invention a aussi pour but la possibilité d'apprécier dans quelle mesure des agents extérieurs peuvent interférer avec l'interaction entre le rétrovirus étudié et des clones cellulaires infectables, grâce à l'influence que ces agents extérieurs exercent sur le susdit marquage.

L'invention met à profit la capacité, déjà observée pour des recombinants rétroviraux contenant un gène exogène dont l'expression est recherchée, à s'incorporer dans le génome de cellules individuelles infectables par le rétrovirus sauvage correspondant et à assurer l'expression du gène exogène, dans les cellules individuelles infectées, ce gène exogène étant transmissible d'une génération cellulaire à l'autre.

L'invention concerne plus particulièrement un rétrovirus recombinant défectif dérivé d'un rétrovirus sauvage ou natif, contenant les parties agissant en "cis" du virus sauvage et assurant normalement l'empaquetage du rétrovirus, sa réverse-transcription en une molécule ADN et son intégration dans le génome de la cellule hôte, ainsi que le site de polyadénylation de cet ARN, ce rétrovirus recombinant étant plus particulièrement caractérisé par la délétion d'au moins une partie sinon de la totalité des séquences codant pour les protéines virales du virus sauvage ou natif, la partie délétée étant suffisamment importante pour que soit interdite la réplication du rétrovirus défectif qui en résulte dans la cellule-hôte, et par la substitution à la partie délétée d'une séquence génique contenant elle-même une séquence codant pour une protéine choisie parmi celles qui peuvent agir en tant que marqueurs des cellules-hôtes infectées, lorsque celles-ci sont infectées par le rétrovirus recombinant défectif ainsi constitué. Les cellules marquées peuvent le cas échéant être isolées, notamment grâce à leur coloration sélective, éventuellement sous l'action ultérieure d'un révélateur.

La partie délétée des séquences codant pour les protéines virales, voire d'autres séquences non essentielles pour la réplication virale notamment dans les conditions qui seront décrites plus loin pour la production du rétrovirus recombinant défectif selon l'invention doit aussi être suffisante pour que l'empaquetage du rétrovirus recombinant défectif produit puisse être réalisé.

La séquence génique susdite comprend éventuellement elle-même un promoteur susceptible d'être reconnu par les polymérases de l'hôte cellulaire infectable (par exemple un promoteur de SV40, lorsque l'hôte cellulaire est constitué par des cellules de mammifères). En variante la séquence génique peut être limitée pour l'essentiel à la séquence codant pour le marqueur, placée sous le contrôle direct du promoteur rétroviral correspondant, notamment de la région LTR5' du rétrovirus. En variante encore, l'une ou l'autre des régions LTR, ou les deux à la fois, sont dépourvues de promoteur(s), la séquence génique codant pour le marqueur étant alors sous le contrôle d'un promoteur distinct.

Par exemple un rétrovirus recombinant défectif conforme à l'invention comprend successivement :

- la région LTR5′ du rétrovirus sauvage correspondant, suivie de la région d'empaquetage ;
- le site d'initiation de l'ARN correspondant aux protéines virales normalement codées par les gènes gag, pol et env ;
- la séquence génique susdéfinie, concernant la séquence codant pour le marqueur, en lieu et place de tout ou partie de la région rétrovirale comportant les gènes gag, pol et env, cette séquence génique étant placée sous le contrôle d'un promoteur approprié, le cas échéant de la région LTR5′ elle-même,
- la région LTR3′ , qui coincide avec le site de polyadénylation de l'ARN.

Le marqueur lui-même est avantageusement constitué par une protéine produite dans les cellules infectées par le virus recombinant défectif, pour lui conférer une coloration particulière ou par toute protéine capable d'agir sur un substrat pénétrant dans les cellules infectées et de subir une modification de coloration, ou plus généralement de son spectre d'absorption des radiations lumineuses, sous l'action de cette protéine. A titre d'exemples de protéines codées par la séquence codante, contenue dans la séquence génique mise en oeuvre dans l'invention, on mentionnera la ß-galactosidase, la ß-glucuronidase ou la luciférase. Il va de soi que l'on peut avoir recours à toute autre séquence codante, dès lors qu'elle code pour une protéine susceptible de jouer le rôle de marqueur permettant de repérer aisément les cellules transformées.

Il est avantageux, pour obtenir un marquage très sensible, de placer le marqueur sous le conrôle d'un promoteur fort, eucaryote, et susceptible d'être reconnu par les cellules étudiées et éventuellement sensibles à l'infection par le rétrovirus sauvage. Par exemple dans le rétrovirus recombinant défini plus haut, le marqueur est placé sous le contrôle d'un promoteur autorisant la rétention du marqueur dans un compartiment cellulaire aisément identifiable des cellules étudiées, l'ensemble formant la séquence génique sus-définie, elle-même placée sous le contrôle de la région LTR5′ du rétrovirus concerné. Une séquence génique avantageuse est ainsi constituée par la séquence "nls-Lac Z" décrite par Kalderon, D. et al (1984), Cell 39, 499-509, et contenant une région signal de localisation nucléaire (nls) de l'antigène T du virus de singe SV40 fusionnée au gène Lac Z. La protéine de fusion correspondante nls-ß-Gal est enzymatiquement active et reste associée avec le noyau des cellules auxquelles une telle séquence génique est incorporée, plus particulièrement avec les membranes des noyaux cellulaires desdites cellules, plutôt que de s'accumuler dans le nucléoplasme.

L'utilisation de tels types de marqueurs dans le cadre de l'invention est particulièrement avantageux, en tant qu'elle permet une observation individualisée des cellules auxquelles les virus ou provirus défectifs recombinants sus-définis ont été incorporés, notamment à l'occasion de la révélation de l'expression du marqueur, par exemple dans les conditions décrites plus loin.

Quelle que soit la construction envisagée, le rétrovirus recombinant défectif selon l'invention est tel qu'il conserve sa capacité d'infecter un hôte cellulaire compétent, cette capacité d'infection étant cependant "épuisée" dès lors que le rétrovirus recombinant a pénétré dans l'hôte cellulaire compétent, faute de disposer des séquences essentielles codant pour les protéines virales du rétrovirus sauvage, qui lui permettraient de s'y propager. Cependant, grâce à la capacité que possède alors le rétrovirus recombinant à s'intégrer sous forme de provirus au génome de la cellule hôte, et à l'expression alors induite du marqueur, les cellules transformées peuvent être aisément détectées, le cas échéant après une opération de révélation postérieure de ce marqueur.

D'une façon générale, le procédé selon l'invention pour la détection du pouvoir infectieux d'un rétrovirus "sauvage" déterminé à l'égard de tout ou partie d'une culture cellulaire est donc caractérisé :

- par la mise en contact de cette culture cellulaire, avec un rétrovirus recombinant défectif tel que défini ci-dessus dans des conditions éventuellement susceptibles d'autoriser cette infection, ce rétrovirus recombinant défectif ayant en commun avec le rétrovirus sauvage les parties agissant en "cis" du virus sauvage (autrement dit les parties directement associés au rétrovirus recombinent) et assurant normalement la transcription virale, l'empaquetage du rétrovirus dans le génome de la cellule hôte, ainsi que le site de polyadénylation de cet ARN, et

- par la visualisation, le cas échéant à l'aide d'un révélateur approprié, de celles des cellules dans lesquelles le marqueur est exprimé.

La détection d'une expression effective du marqueur manifeste alors la capacité que possède aussi le rétrovirus sauvage correspondant d'infecter lesdites cellules ou des cellules du même type, dès lors qu'elles seraient exposées à ce rétrovirus recombinant défectif, tandis que la non-expression du marqueur dans aucune des cellules traitées autorise à conclure à la vraisemblable absence de pouvoir infectieux du rétrovirus sauvage correspondant à l'égard des cellules du même type.

Une variante de ce procédé peut être mise en oeuvre pour l'étude de la capacité éventuelle d'agents extérieurs à interférer avec la manifestation du pouvoir infectieux d'un rétrovirus vis-à-vis d'une culture cellulaire sensible à ce rétrovirus. Cette variante consiste dans l'application du procédé défini ci-dessus mettant en jeu le rétrovirus recombinant défectif, cette fois-ci à l'égard de cultures de cellules dont est connue la sensibilité à ce rétrovirus, et ce en présence de l'agent extérieur, par exemple un principe actif possible de médicament dans le milieu de culture desdites cellules. La capacité de cet agent extérieur à éventuellement interférer avec l'interaction entre le rétrovirus et lesdites cultures sensibles peut alors être appréciée selon le degré d'expression observé, comparé à celui obtenu à l'issue de la mise en oeuvre du même procédé, mais en l'absence de l'agent extérieur étudié.

Celui-ci est d'autant plus efficace que la dose utilisée conduisant à l'inhibition de l'expression détectable du marqueur dans les conditions de l'essai est plus faible.

L'invention concerne aussi les nécessaires ou kits réunissant :

- des rétrovirus recombinants défectifs, tels que ci-dessus définis (ou les moyens permettant de les produire à la demande, tels qu'ils seront envisagés plus loin),
- le révélateur, notamment un substrat de l'enzyme consistant en le produit d'expression du marqueur de ce rétrovirus recombinant,
- le cas échéant, les tampons et réactifs nécessaires à la réalisation de la susdite mise en contact de ce rétrovirus recombinant avec la culture cellulaire dont est recherchée la sensibilité à l'infection par le rétrovirus sauvage correspondant, et ce en présence ou non d'agents extérieurs, tels que principes actifs de médicaments susceptibles d'interférer avec la manifestation du pouvoir infectieux du rétrovirus vis-à-vis de ces cellules sensibles.

L'invention concerne également les vecteurs recombinants contenant l'ADN du virus recombinant défectif sus-défini, incorporé à tout vecteur permettant l'amplification de l'ensemble dans un hôte cellulaire compétent, par exemple un plasmide bactérien.

L'invention concerne également un procédé de fabrication du rétrovirus recombinant défectif selon l'invention, ce procédé comprenant :

- la transfection avec un vecteur recombinant défectif tel que défini ci-dessus, d'une lignée cellulaire auxiliaire contenant, incorporée à son propre ADN génomique, une séquence nucléique (ci-après dénommée "séquence de complémentation") capable de suppléer à l'absence dans le vecteur recombinant défectif des séquences, codant pour les protéines virales autorisant sa propagation dans les cellules qu'il est susceptible d'infecter. En particulier la séquence de complémentation contient les parties essentielles des gènes <u>gag</u>, <u>pol</u> et <u>env</u> du rétrovirus sauvage correspondant, assurant un apport "en trans" (autrement dit un apport extérieur) des séquences de nucléotides codant pour les protéines virales permettant la réplication, dans les cellules de cette lignée auxiliaire, d'un virus recombinant défectif contenant dans son génome les autres séquences rétrovirales du vecteur sauvage ;
- la culture de la lignée cellulaire auxiliaire ainsi transfectée dans des conditions permettant la réplication de ce rétrovirus recombinant <u>in situ</u>, grâce à l'apport par complémentation "en trans" des protéines virales codées par la susdite "séquence de complémentation" ;
- la récupération du virus recombinant défectif produit dans le surnageant de culture de ladite lignée cellulaire auxiliaire.

De préférence la séquence de complémentation contient plus particulièrement la partie de l'ADN génomique du rétrovirus sauvage correspondant, codant notamment pour les protéines virales, à savoir les gènes <u>gag</u>, <u>pol</u> et <u>env</u> du rétrovirus sauvage correspondant, dont le vecteur recombinant défectif est lui-même dépourvu.

Cependant il n'est pas nécessaire que la "séquence de complémentation" coïncide toujours exactement avec une partie du rétrovirus sauvage, dont est également issu l'ADN rétroviral contenu dans le vecteur recombinant. Séquence de complémentation et ADN rétroviral peuvent également être issus de rétrovirus voisins présentant entre eux le degré de compatibilité requis, pour que l'apport en trans des protéines virales puisse s'opérer.

Avantageusement la lignée cellulaire utilisée contient, sous forme de provirus, la totalité du rétrovirus sauvage correspondant, dont cependant les séquences d'empaquetage ont été délétées.

L'invention concerne encore les nécessaires ou kits permettant la production "à la demande" du rétrovirus recombinant conforme à l'invention, les éléments essentiels de ces kits consistant :

- en un vecteur recombinant contenant le génome du rétrovirus conforme à l'invention ;
- en un hôte cellulaire auxiliaire susceptible d'être transfecté dans les conditions qui ont été décrites, pour produire le rétrovirus recombinant lui-même ;

auxquels s'ajoutent éventuellement

- des tampons et réactifs nécessaires à la réalisation de la susdite transfection ;
- et, le cas échéant, un hôte cellulaire compétent, notamment bactérien, permettant une production amplifiée du susdit vecteur recombinant.

L'invention concerne naturellement aussi les kits, tels que ceux qui ont été envisagés plus haut et dans lesquels le rétrovirus recombinant défectif est remplacé par l'ensemble des constituants du dernier kit ci-dessus, pour la fabrication à la demande du rétrovirus recombinant défectif.

Le procédé selon l'invention de production de rétrovirus recombinant défectif décrit ci-dessus permet aussi, grâce à la complémentation qu'assure la séquence de oomplémentation, de suppléer au caractère défectif du gènome du virus recombinant introduit dans le génome des cellules de la lignée auxiliaire et de permettre la réplication, la production et l'excrétion par cette lignée auxiliaire d'un virus recombinant défectif, lequel peut à son tour être utilisé comme virus infectieux - une fois -à l'égard d'une culture cellulaire à étudier, soit que l'on veuille détecter la capacité de cette culture cellulaire à être infectée par le rétrovirus correspondant, voire identifier celles des cellules qui au sein de la culture cellulaire sont susceptibles d'être infectées, soit que l'on veuille évaluer l'action de substances éventuellement inhibitrices de la capacité du rétrovirus sauvage correspondant à l'infecter, dès lors que la sensibilité de cette culture cellulaire à ce rétrovirus aurait auparavant été reconnue.

On observe que l'hôte cellulaire auxiliaire peut également contenir, incorporé à son ADN génomique, la totalité du rétrovirus sauvage à l'état de provirus. L'utilisation d'un tel hôte cellulaire sera cependant susceptible d'entraîner la production par celui-ci, du rétrovirus sauvage parallèlement à celle du rétrovirus recombinant selon l'invention. Le mélange de rétrovirus ainsi obtenu pourra également être utilisé pour la réalisation des essais sus-décrits, dès lors que le rétrovirus natif infectieux ne s'opposera pas au marquage des cellules infectées par le rétrovirus recombinant défectif dans les conditions qui ont été indiquées. La réalisation des essais pourra néanmoins s'en trouver compliquée, surtout dans le cas où le rétrovirus sauvage ou natif présente un caractère fortement pathogène.

Cette dernière technique fournit néanmoins, en variante, un procédé de détection (ou de diagnostic in vitro) de l'existence ou non d'un virus, comprenant les gènes gag, pol etenv dans des cultures cellulaires ou prélèvements de fluides biologiques provenant de patients et susceptibles de le contenir, dès lors que ces cultures cellulaires sont amenées à jouer le rôle du susdit hôte cellulaire auxiliaire vis-à-vis du rétrovirus recombinant. Le procédé selon l'invention est d'un intérêt tout particulier pour le diagnostic d'une infection virale par un virus HIV, chez un sujet à risques éventuellement infecté par un virus HIV, mais dont le sérum se sera révélé ne pas encore contenir des anticorps anti-HIV (abréviation de l'expression anglaise "Human Immunodeficency Virus") (Virus Humain d'Immunodéficience, aussi dénommé LAV ou HTLV-III) et qui contient donc des cellules infectées et des cellules non infectées.

L'infection de tels mélanges de lymphocytes par un vecteur recombinant conforme à l'invention, dérivé de HIV, ne pourra en effet s'accompagner de la production du rétrovirus recombinant que sous réserve que certains de ces lymphocytes contiennent HIV à l'état de provirus. En l'absence de provirus, et par conséquent des gènes gag, pol, env et pX correspondants, aucune complémentation, et par conséquent aussi aucune production de virus recombinant ne pourait être observée. Au contraire, l'observation d'une production du virus recombinant conforme à l'invention conduirait à la conclusion que l'individu dont provenait le prélèvement avait été infecté par le virus HIV. La détection du virus recombinant implique naturellement aussi, dans ce dernier cas, la mise en contact du surnageant de culture avec des cellules sensibles à l'infection virale et par révélation de l'expression du marqueur dans les cellules sensibles infectées.

L'invention concerne aussi plus particulièrement les cultures cellulaires infectées par le rétrovirus recombinant défectif selon l'invention ou encore les cultures cellulaires contenant le provirus recombinant défectif correspondant, notamment en tant que réactifs pour la détection du rétrovirus sauvage correspondant ou d'un rétrovirus apparenté dont l'ADN comprend des gènes gag, pol et env et qui, lorsqu'il pénètre dans les susdites cultures cellulaires apparentées, permettent aussi la réplication du virus recombinant défectif.

En d'autres termes, l'invention concerne également, selon l'une de ses variantes supplémentaires, un procédé de détection d'une infection rétrovirale dans un échantillon contenant des cellules (tel qu'un prélèvement de sérum humain contenant des lymphocytes T4, s'agissant d'apprécier s'il est infecté par un virus HIV) ou du milieu, tel qu'un surnageant de culture de ces cellules, auparavant au contact desdites cellules, ce procédé étant caractérisé par les étapes que constituent :
- la mise en contact de cet échantillon avec les cellules contenant un rétrovirus ou prophage défectif susceptible d'être lui-même complémenté par l'apport extérieur en rétrovirus éventuellement contenu dans l'échantillon, et ce dans des conditions (notamment de température et de durée d'incubation appropriées) permettant l'infection des cellules contenant le rétrovirus ou provirus recombinant défectif par le rétrovirus éventuellement présent dans l'échantillon, la réplication et la production alors induite du rétrovirus défectif correspondant dans le milieu d'incubation obtenu,
- la mise en contact du milieu d'incubation précédent, de préférence le surnageant de ce milieu, avec des cellules non infectées (cellules saines), néanmoins sensibles à l'infection par le rétrovirus sauvage, et ce dans des conditions permettant l'infection éventuelle desdites cellules saines placées au contact de ce milieu d'incubation ou de ce surnageant, et
- la détection de l'infection éventuelle desdites cellules antérieurement saines, par révélation de l'expression du marqueur incorporé au rétrovirus recombinant défectif, alors éventuellement présent dans ces dernières cellules, à l'aide d'un révélateur approprié.

Un tel procédé permet la détection très sensible, dans des délais très rapides, de la présence ou non d'une infection rétrovirale dans l'échantillon cellulaire initial qui était à tester, et même le dosage du degré de l'infection rétrovirale dans l'échantillon testé, au moment où le test a été réalisé.

Le procédé selon l'invention, qui permet une détection précoce et ultrasensible plus particulièrement des virus HIV dans un échantillon biologique, est d'autant plus intéressant que les tests de séro-positivité mettant en oeuvre la détection d'anticorps contre le rétrovirus HIV chez les porteurs éventuels peuvent s'avérer insuffisants dans le stade précoce de l'infection rétrovirale. Il a en effet été observé que les anticorps recherchés n'apparaissent souvent chez un sujet infecté par le virus HIV qu'après une durée d'incubation de 12 à 18 mois. D'autres tests actuellement utilisés, tels que la détection et le dosage de l'activité réverse transcriptase du virus éventuellement présent dans les prélèvements étudiés ne présentent pas toujours le degré de sensibilité voulue pour conclure utilement à l'infection ou non du sujet dont provenait le prélèvement.

On appréciera que la procédé selon l'invention permet aussi d'étudier l'évolution de l'infection chez un patient atteint de la maladie induite par le rétrovirus sauvage, s'il est répété dans le temps, sur des prélèvements de sérums ou de tout autre fluide biologique infecté provenant de ce patient.

Comme dans le cas précédent, la dernière variante de procédé décrite peut être mise en oeuvre pour l'étude de l'efficacité éventuelle d'un principe actif de médicament vis-à-vis de l'interaction entre le rétrovirus considéré et des cellules sensibles, notamment lorsque la première mise en contact sus-indiquée est opérée en présence de ce principe actif. On apprécie que le test ainsi modifié permet à la fois de sélectionner le principe actif de médicament qui pourrait se révéler le plus actif, de déterminer les doses qui pourraient être efficaces pour traiter le patient, ces doses efficaces découlant notamment de celles qui, dans la mise en oeuvre du test sus-indiqué, inhibent l'infection des cellules contenant le rétrovirus ou provirus recombinant défectif au contact de prélèvements provenant du malade à traiter ou le surnageant du milieu dans lequel cet échantillon cellulaire avait été maintenu.

L'invention concerne donc également des nécessaires ou kits permettant la mise en oeuvre du procédé qui vient d'être décrit, ces kits comportant :

- des cellules sensibles modifiées contenant le provirus recombinant défectif sus-défini,
- des cellules semblables mais "saines" en ce qu'elles ne sont infectées ni par le rétrovirus recombinant défectif, ni par le rétrovirus sauvage,
- éventuellement les tampons et réactifs nécessaires à la réalisation, d'une part, d'une mise en contact des cellules sensibles modifiées avec l'échantillon biologique à étudier et l'incubation du mélange de réaction dans des conditions permettant l'infection éventuelle des cellules sensibles modifiées par l'échantillon étudié et, d'autre part, d'une mise en contact des cellules saines avec les rétrovirus recombinants défectifs éventuellement libérés dans le milieu d'incubation des cellules sensibles modifiées infectées, après leur mise précédente en contact avec un prélèvement infecté par le rétrovirus à détecter,
- le cas échéant des milieux de conservation ou de culture pour lesdites cellules saines et modifiées, et
- des moyens de révélation, notamment un substrat de l'enzyme susceptible d'être exprimée par le marqueur incorporé au rétrovirus ou provirus recombinant défectif susdit.

Avantageusement lesdites cellules sensibles modifiées et lesdites cellules "saines" utilisées dans ces kits sont constituées par des lignées cellulaires "immortalisées" portant les récepteurs permettant leur infection par le rétrovirus. Par exemple, dans le cas d'un kit destiné à la détection d'un virus HIV, lesdites cellules sensibles modifiées et les cellules saines appartiendraient toutes deux à des lignées cellulaires du type CEM, HUT, etc....

Dans les exemples indiqués plus loin, les possibilités de l'invention sont illustrées en rapport avec la production d'un virus recombinant défectif dérivé du Virus Moloney de la Leucémie Murine (M-MuLV ; abréviation du nom anglais du même virus : "Moloney Murine Leukemia Virus"). En particulier une production d'un rétrovirus recombinant correspondant sera décrite cette production impliquant alors la transformation d'une lignée auxiliaire, constituée de cellules ω2, telles que décrites par Mann et al (1983), Cell, 33, 153-159. Cette lignée dérivée de cellules de souris contient, intégrée dans son propre génome, la totalité de l'ADN génomique de M-MuLV, à l'exception de la région d'empaquetage du rétrovirus qui avait été délétée. L'article de Mann et al décrit également un procédé pour incorporer cet ADN génomique, pourvu de ces mêmes délétions, dans le génome de lignées cellulaires normalement infectables par M-MuLV. Il est à remarquer transposable à la production de toute autre lignée cellulaire contenant, incorporée à son génome, la totalité du génome d'un autre rétrovirus, dont la région d'empaquetage du génome rétroviral aura au préalable été délétée, dès lors que les conditions suivantes sont remplies. D'une part, la capacité de cette autre lignée cellulaire à être infectée par le rétrovirus sauvage correspondant a été reconnue et, d'autre part, la structure du génome de cet autre rétrovirus a été suffisamment élucidée pour que sa région d'empaquetage ait été localisée et délétée, notamment grâce à l'utilisation des enzymes de restriction appropriées, mises en oeuvre dans des conditions bien connues de l'homme de métier. Tel est, entre autres, le cas pour les autres rétrovirus identifiés ci-après à titre d'exemples.

Le procédé qui vient d'être décrit ne saurait être considéré comme limitatif, en ce qui concerne la production du rétrovirus recombinant défectif. On peut aussi avoir recours à la transformation des cellules sensibles par un vecteur approprié modifié par un insérat consistant en un cADN dérivé du génome entier du rétrovirus recombinant défectif dont la production est à faire, selon des techniques devenues classiques dans le domaine du génie génétique.

Il est tout à fait clair que l'invention s'adresse à la détection de tout type de rétrovirus éventuellement infectieux vis-à-vis de cultures cellulaires déterminées et présentant les caractéristiques essentielles de structure dont il a été question plus haut. En particulier, elle s'applique à la fabrication de rétrovirus recombinants défectifs permettant, dans les conditions qui ont été indiquées, la détection du pouvoir infectieux (ou la modulation du pouvoir infectieux) des rétrovirus connus sous les dénominations HTLV I, HTLV II, HIV I (ou encore LAV ou HTLV III), HIV II (ou LAV II), HTLV IV et de nombreux rétrovirus infectieux à l'égard de l'animal. A titre d'exemple, on mentionnera les rétrovirus leucémiques des bovins (Bovine Leukemia Viruses) et des félins (FeLV).

Une variante du procédé selon l'invention pour l'étude de l'activité possible de principes actifs de médicaments consiste à infecter une souris infectable par le rétrovirus recombinant et par le rétrovirus sauvage correspondant (par exemple ceux correspondant à MMuLV, de telle sorte que la souris produise en continu le rétrovirus recombinant comportant le marqueur, notamment LacZ. L'animal peut alors servir de modèle in vivo pour l'ensemble des procédés décrits in vitro, notamment pour ce qui est de l'étude de l'activité possible de principes actifs de médicaments contre des rétrovirus en général. Un médicament se révèlera actif si la proportion des cellules marquées chez la souris traitée est réduite par rapport à des souris "saines" témoins, également traitées par le même médicament.

Grâce à se capacité d'incorporation dans le génome des cellules qu'il infecte, le rétrovirus recombinant défectif selon l'invention peut également, lorsque cette incorporation est stable et transmissible au cours des générations, être utilisé pour des études de généalogie cellulaire. Ces études revêtent un intérêt particulier, lorsqu'elles sont appliquées à des cultures cellulaires susceptibles de se différencier. Il importe alors naturellement que soit conservée la capacité d'expression du marqueur au cours de cette différentiation. Le cas échéant, pour s'en assurer, on aura recours aux techniques décrites dans la demande de brevet européen n 85.401914.8/0178220 déposée le 1er octobre 1985.

Des caractéristiques supplémentaires de l'invention apparaîtront encore au cours de la description de l'un de ses exemples de réalisation, appliquée à la production d'un rétrovirus recombinant conforme à l'invention, dérivé de M-MuLV. Les techniques décrites à titre d'exemple peuvent être considérées comme représentatives de celles qui pourraient être mises en oeuvre pour la détection du pouvoir infectieux de tout autre type de rétrovirus à l'égard de cellules déterminées, dans des conditions également déterminées ; ou encore pour l'étude de la modulation par un principe actif de médicament de l'interaction entre un rétrovirus et des cultures cellulaires sensibles à l'infection par ce rétrovirus, par exemple entre le virus HIV et des lymphocytes humains à spécificité T4.

D'autres buts de l'invention et les moyens qui permettent de les atteindre résulteront encore de la description de certains de ses modes de réalisation présentés, soit à titre préféré, soit à titre d'illustration des possibilités offertes par l'invention pour l'étude de tous les aspects des diverses interactions possibles entre des rétrovirus déterminés et les cellules ou hôtes infectables par ce rétrovirus et de leur modulation.

Il sera dans ce qui suit fait référence aux figures dans lesquelles :
- la fig. 1 est une représentation schématique d'un plasmide recombinant conforme à l'invention ;
- la fig. 2 concerne des résultats d'analyse en gel d'électrophorèse et par hybridation avec des sondes appropriées de certaines des séquences contenues dans ce plasmide ;
- la fig. 3 montre la corrélation entre les longueurs des séquences dont les tailles résultent de la fig. 2 avec les tailles qui pouvaient être prédites des fragments de restriction par certaines enzymes ;
- la fig. 4 fournit des résultats de l'activité rétrovirale de rétrovirus recombinants conformes à l'invention, produits par des cultures de cellules auxiliaires, dans les conditions qui ont été définies plus haut ;
- les fig. 5a et 5b sont des représentations schématiques de constructions de cADNs de rétrovirus recombinants défectifs dérivés de HIV-1 et

les fig. 6A-6D illustrent de façon schématique un cycle complet de détection d'un rétrovirus sauvage mettant en oeuvre des cellules sensibles contenant un rétrovirus ou prophage recombinant défectif susceptible d'être complémenté en trans par l'apport extérieur de rétrovirus sauvage.

## Exemple I

### Production d'un rétrovirus recombinant :

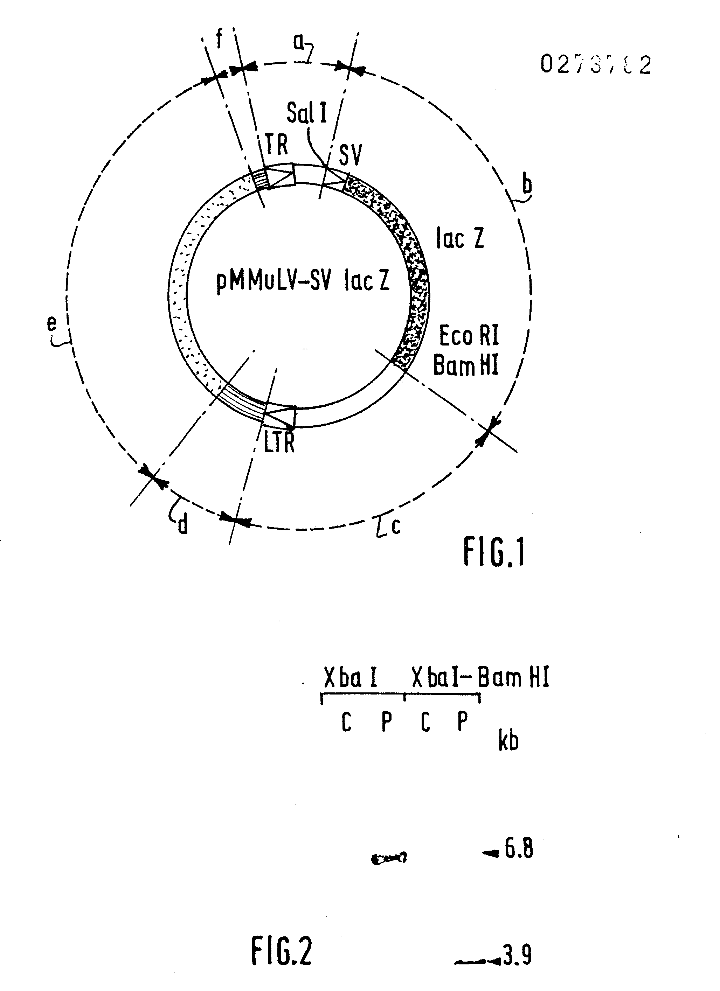

Le plasmide pMMuLV-SV-lac Z (fig. 1a) a été construit en ayant recours aux techniques décrites par Maniatis et al. (1982), Cold Spring Harbor, N.Y., et ce en utilisant des enzymes de restriction fournies par BOEHRINGER-MANNHEIM.

Cette fabrication a été réalisée à partir de trois fragments.

Le premier fragment, contenant les séquences bactériennes (dérivées de pBR322) et les séquences rétrovirales, a été obtenu à partir du plasmide pB6 décrit par P. Yermanian (1984) "Dissertation" (Université Paris XII, Paris) et contenant le génome proviral de M-MuLV (Harbors et al. (1981), Proc. Natl. Acad. Sci. USA, 78, 7609-7613. Ce premier fragment consistait donc en fait dans le fragment de 8,2 kb qui a été isolé à partir de pB6, après digestion de ce plasmide avec SalI et BamHI.

Le second fragment, contenait le promoteur SV40 et une partie du gène de E. coli lacZ. Ce second fragment a été isolé à partir du plasmide pCH110 décrit par Hall et al. (1983), J. Mol. Appl. Genet. 2, 101-109, si ce n'est qu'un site de restriction SalI avait au préalable été inséré, dans son site PVUII, à proximité de l'extrémité 5' du promoteur SV40. Le second fragment consistait plus particulièrement dans le fragment de 3,6 kb isolé à partir de ce plasmide, après digestion de celui-ci avec SalI et EcoRI.

Le troisième fragment qui contenait une région d'extrémité 3' du gène lacZ a été obtenu à partir d'un plasmide pMLB524, décrit par Parsot et al. (1982), Mol. Gen. Genet., 188, 455-458, si ce n'est que le site AvaI de ce plasmide avait auparavant été transformé en un site BamHI. Ce troisième fragment de 320 paires de bases a été obtenu à partir du plasmide pMLB524 modifié comme indiqué ci-dessus, par digestion de celui-ci avec BamHI et EcoRI.

Ces trois fragments ont été ligués pour fournir le plasmide pMMuLV-SV-lacZ, représenté à la fig. 1 et dans lequel :
- les parties a et c proviennent de M-MuLV ;
- la partie b comprend un promoteur SV40 (SV sur le dessin) suivi d'une séquence lacZ de E. coli ;
- les séquences d et f proviennent d'un ADN génomique de souris, lesquelles étaient initialement contenues dans pB6 et, enfin,
- la séquence e provient de pBR322.

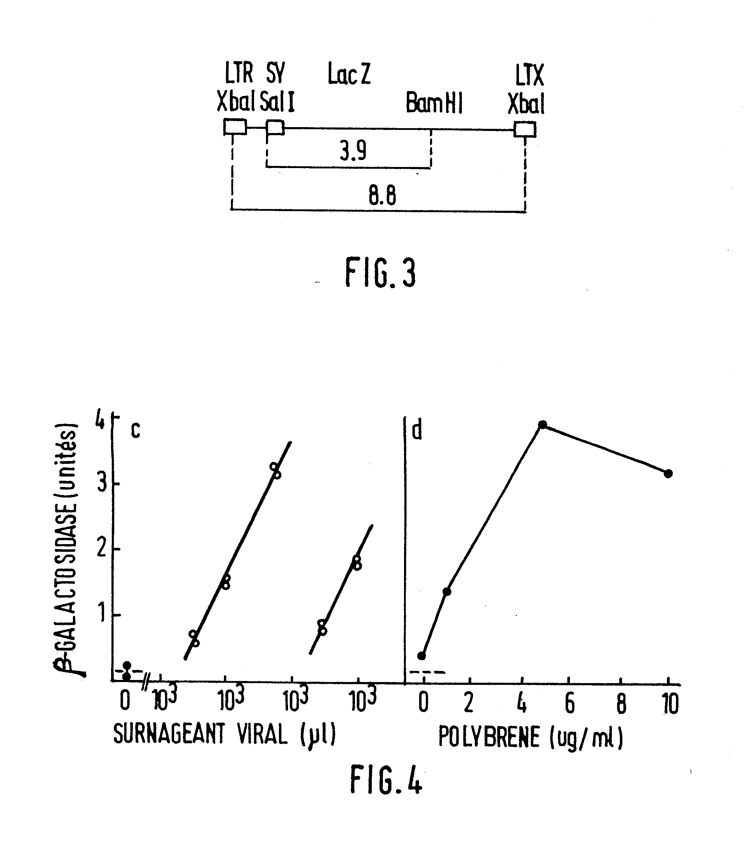

L'analyse des éléments de structure du plasmide recombinant obtenu, d'une part, et d'un provirus incorporé dans des cellules NIH 3T3, suite à leur infection avec un rétrovirus recombinant ultérieurement produit à l'aide de ce plasmide (voir ci-après), d'autre part, résulte des fig. 2 et 3. Cette analyse a été faite par digestion de ce plasmide et de ce provirus à l'aide des enzymes de restriction Xbal, d'une part, et Sall et BamHI, d'autre part, et par électrophorèse sur gel d'agarose. Une sonde d'hybridation consistant en un fragment SV-lacZ purifié, a été utilisée pour identifier les fragments portant des séquences communes.

Les distances relatives de migration des fragments hybridant dans le technique dite du "Southern blot" (buvard de Southern) et respectivement issus du plasmide (bandes de gel marquées d'un "P") et des fragments issus du provirus (bandes de gel marquées d'un "C") apparaissent dans la fig. 2.

Les tailles de ces fragments, telles qu'elles découlent de leurs distances relatives de migrations observées vis-à-vis des distances de migration de molécules témoins de poids moléculaires connus, coïncident sensiblement avec les tailles des fragments rétroviraux Sall-BamHI et Xbal-Xbal correspondants contenus dans le vecteur recombinant (fig. 3).

Production d'un rétrovirus recombinant conforme à l'invention :

Le rétrovecteur recombinant décrit ci-dessus a été utilisé pour la production d'un rétrovirus recombinant conforme à l'invention dans une lignée de cellules auxiliaires de souris Ψ2 contenant un génome de provirus M-MuLV dont la région fournissant normalement un signal d-empaquetage avait été délétée. Cette lignée ne produit pas de virus sauvage.

Les cellules Ψ2 ont été transfectées simultanément avec le rétrovirus recombinant sus-décrit et avec un plasmide pSVtkneoβ (Nicolas et al. (1983), eds. Silver, L.M., Martin, G.R. et Strickland, S., Cold Spring Harbor Conferences on cell proliferation, 10, 469-485, Cold Spring Harbor Laboratory, Cold Spring Harbor, N.Y.) utilisé à titre de marqueur eucaryote. Le plasmide pSVtkneoβ contient un gène codant pour une enzyme conférant aux cellules une résistance à l'aminoglycoside G418, ce plasmide permettant par conséquent de sélectionner des clones co-transformés par les deux plasmides dans un milieu de culture contenant du G418, décrit par Colbère-Garapin et al. (1981), J . Mol. Biol. 150, 1-14, ces clones co-transformés se révélant à la fois résistants au G418 et capables d'exprimer la β-galactosidase (cellules β-galactosidase positives).

Les différents clones β-galactosidase positifs ont été testés pour leur capacité à produire un rétrovirus recombinant conforme à l'invention en procédant comme suit. Les surnageants de ces clones ont été introduits dans le milieu de culture de cellules de souris NIH 3T3 et les cellules ayant incorporé le rétrovirus ont été détectées grâce à leur capacité à exprimer une activité β-galactosidase. Le clone meilleur producteur de rétrovirus recombinant, dénommé LZ1, a été isolé et son ADN a été étudié, par analyse de restriction. Il s'est révélé contenir une structure de provirus ne différant pratiquement pas de la structure rétrovirale contenue dans le rétrovecteur recombinant initialement mis en oeuvre.

Infection unique de cellules infectables par le rétrovirus recombinant :

Toute méthode appropriée peut être mise en oeuvre pour détecter l'expression de la séquence lacZ. Une méthode avantageusement utilisée est la méthode de coloration histochimique de la β-galactosidase selon Lojda (1970), Histochemie, 23, 266-288, pour détecter l'expression de lacZ dans des cellules infectées par le virus. On peut notamment procéder comme suit. Les cellules sont rincées avec une solution NaCl 150 mM, de phosphate de sodium 15mM et tamponnées à pH 7,3 avec le tampon PBS. Les cellules sont ensuite fixées pendant 5 minutes à 4 C dans une solution de PBS contenant 2 % de formaldéhyde et 0,2 % de gluteraldéhyde. Les cellules sont ensuite lavées avec du PBS et recouvertes avec le milieu de réaction histochimique contenant 1 mg/ml de 4-Cl-5-Br-3-indolyl-β-D-galactoside (X-Gal—, 5 mM de ferricyanure de potassium, 5mM de ferrocyanure de potassium et 2 mM MgCl₂. Le X-Gal est dissous dans une solution de 40 mg/ml de diméthoxysulfoxide (DMSO), avant d'être introduit dans le milieu de réaction. Le cas échéant 1 % d'agar peut être ajouté au milieu de culture pour minimiser le détachement des cellules de leur support. L'incubation est en général conduite à 37°C pendant 14-18 heures.

C'est une telle méthode qui a été utilisée pour repérer les cellules NIH 3T3 qui avaient été infectées.

A l'issue de la mise en oeuvre de ce test, les cellules ayant incorporé le provirus comportant lui-même le gène lacZ apparaît fortement coloré vis-à-vis des cellules dont les génomes n'ont pas été modifiés.

Le même test de détection peut être mis en oeuvre dans les essais de détection des cultures cellulaires qui, dans des conditions déterminées, peuvent ou non être infectées par le rétrovirus recombinant selon l'invention.

Essai d'infection de cellules déterminées par le rétrovirus recombinant selon l'invention :

Les conditions dans lesquelles une telle infection peut être réalisée ont déjà été partiellement évoquées ci-dessus.

Il est méanmoins avantageux, pour la sensibilité du test, de concentrer au préalable le rétrovirus recombinant produit par les cellules auxiliaires, par exemple par centrifugation ou filtration, le sédiment ou le produit retenu sur le filtre étant ensuite remis en suspension dans un petit volume du milieu de culture, complété avec du sérum de veau. Par exemple, le sédiment ou le virus retenu sur le filtre est remis en suspension dans un volume de 0, 2 à 2 % de celui du milieu de culture dans lequel il avait initialement été libéré.

Les cellules à tester peuvent alors être exposées soit au surnageant des cellules auxiliaires ayant produit le rétrovirus recombinant ou à un concentrat de rétrovirus. Les cellules éprouvées sont alors ensuite soumises au test de détection par coloration décrits ci-dessus deux jours plus tard. Il est avantageux de réaliser les opérations qui précèdent en présence de polybrène. Ce composant favorise le titre en virus produit.

La fig. 4 fait apparaître les accroissements relatifs d'activité β-galactosidase mesurés (unités β-galactosidase) selon que l'on opère avec un surnageant viral non concentré (courbe de droite de la moitié gauche de la figure) ou concentré (courbe de gauche de la moitié gauche de la figure).

La partie droite de la fig. 4 fait apparaître la variation d'activité β-galactosidase mesurable selon la concentration en polybrène du milieu.

En apparence, en présence de 5 à 10 µg/ml de polybrène, on multiple le titre infectieux apparent par un facteur supérieur à 10. Les unités d'activité β-galactosidase par mg de protéine cellulaire sont telles que définies par Miller J. (1972), Cold Spring Harbor Laboratory, Cold Spring Harbor, N.Y..

Utilisation des rétrovirus LacZ pour détecter une substance antivirale : Exemple de l'AZT.

Pour illustrer encore comment l'invention peut servir à détecter des drogues antivirales, l'expérience suivante a été réalisée. Une concentration variable de l'azidothymidine (BWA509U, réf.PNAS, vol. 83, p. 8333), connue pour inhiber le virus du SIDA HIV-1 (même référence) a été mélangé à $4.10^3$ virus LacZ et mis sur les cellules 3T3 dans les conditions précédemment décrites. Alors que 6448 cellules exprimant le virus ont été détectées dans une expérience témoin, mettant ainsi en oeuvre $4.10^3$ virus LacZ en l'absence d'AZT, aucune des cellules maintenues au contact de l'AZT ne s'est révélée exprimer le gène LacZ jusqu'à une dilution de $10^{-6}$ M en AZT dans le milieu. Les résultats apparaissent dans le tableau 1 ci-après.

## TABLEAU 1

| Concentration finale de l'AZT (M) | $10^{-4}$ | $10^{-5}$ | $10^{-6}$ | $10^{-7}$ | O |
|---|---|---|---|---|---|
| Nombre de virus LacZ résistant au traitement | O | O | O | 72 | 6448 |

Ce tableau montre les conditions dans lesquelles l'efficacité d'un médicament contre des rétrovirus peut être démontrée.

Extension des constructions LacZ à d'autres virus que MuMLV : exemple du virus humain HIV-1

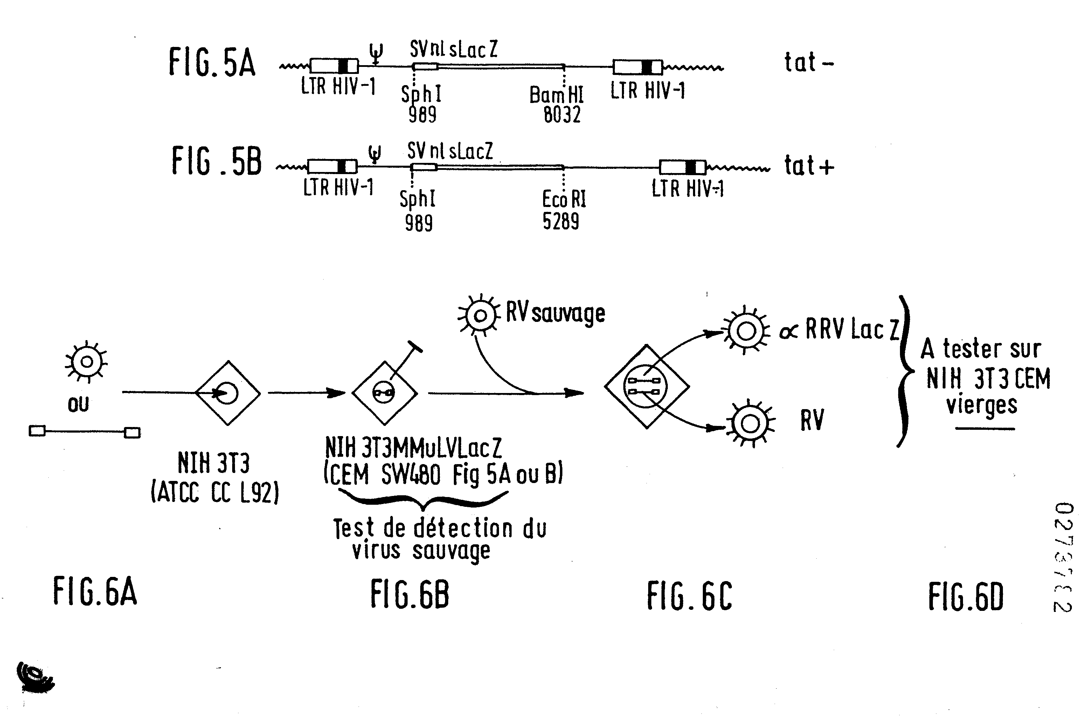

Un virus HIV-1 LacZ peut être construit en suivant les mêmes principes, mais en partant du clone infectieux pNL4-3 d'ADN décrit par Malcom Martin dans Journal of Virology, 59, 284-291 (fig. 1). On peut utiliser le fragment délimité par des sites Sph1 (989éme paire de bases de la séquence décrite dans "RNA Tumor Virus", tome 2, Cole Spring Harbor 1985, p. 1104 et 1105) et BamHI (8032éme paire de bases, même référence) ou EcoRI (5 289éme paire de bases). Ces fragments (fig. 5A et 5B) contiennent les LTR et leurs régions adjacentes. Le gène SV-nlsLacZ (fragment SalI à BamH1 de la fig. 1 pMMuLVSVnls LacZ a été mis à la place des séquences codant gag, pol et env en utilisant les techniques bien connues de l'homme du métier (MANIATIS déjà cité). La plasmide ainsi obtenu possède alors toutes les caractéristiques requises pour être produit par toute lignée humaine infectée par un virus HIV et en particulier par les cellules SW480 (ATCC CCL228) ou CEM (CNCM I-416 et I-417). Le surnageant obtenu à partir de ces lignées infectées est susceptible de contenir des virus recombinants HIV-LacZ utilisables dans les mêmes conditions que les virus MLV-LacZ sus-décrits. Une variante intéressante est décrite dans la fig. 5B où l'expression du gène tat (région Q, de Wain-Hobson) est conservée. Son intérêt n'échappera pas à l'homme du métier pour l'étude des drogues agissant au niveau de l'interaction tat/ARN.

En revanche dans la variante de rétrovirus recombinant défectif représenté à la figure 5A, la séquence tat codant pour une protéine de trans-activation de la région R du génome de HIV a été délétée également. Le provirus dérivé d'un tel rétrovirus défectif peut être doublement activé, lorsque les lignées cellulaires qui le contiennent sont placées au contact d'un échantillon porteur du rétrovirus sauvage, grâce :

1• à la trans-complémentation du provirus par les séquences gag, pol et env du rétrovirus sauvage dans les conditions sus-indiquées ;

2• à la trans-activation de la région R résultant de l'apport extérieur de l'activateur codé par la séquence tat du rétrovirus sauvage.

Utilisation des lignées obtenues par introduction des rétrovirus recombinant lacZ± dans des lignées infectables par les virus sauvages pour en détecter la présence dans tout échantillon biologique.

Il a été expliqué comment le virus défectif MMuLVSV LacZ est produit à l'aide des lignées transcomplémentantes (par exemple des lignées NIH 3T3 (ATCC-CCL92) fig. 6A), d'autres détails peuvent

9

encore être trouvés dans la revue NICOLAS, RUBENSTEIN, éditée par RAYMOND RODRIGUEZ et DT DENHARDT, intitulée "Vectors : a survey of molecular cloning vectors and their uses" (Vecteurs : une revue des vecteurs de clonage moléculaire et leurs utilisations) édité par Butterworth Publishing Company en septembre 1987.

Si on met en présence une lignée de cellules où le virus M-MuLVSV-LacZ aura au préalable été introduit ou un ADN contenant l'information génétique de ce virus (fig. 6B) et un échantillon contenant éventuellement le virus sauvage (RVRs, RVR étant l'abrévation de rétrovirus recombinant), celui-ci agira comme facteur de transcomplémentation, de telle manière que des particules virales ayant empaqueté du RVR-LacZ sont produites dans le surnageant de culture (fig. 6C).

La cellule LacZ$^+$ permet donc de détecter la présence de virus sauvage si l'on révèle le virus RVR- LacZ produit à l'étape C (fig. 6D) par la technique précédemment décrite, à savoir la mise en contact du surnageant de culture de ces lignées LacZ$^+$ infectées, avec des cellules NIH-3T3 vierges (initialement exemptes de rétrovirus recombinant défectif) et révélation de l'incorporation dans celles-ci du marqueur Lac-Z.

Il n'échappera pas à l'homme de métier que ce test s'applique en particulier à toute cellule humaine infectable par les virus HIV, si cette cellule a intégré dans son génome une construction du type décrit dans les fig. 5A ou 5B, ou toute autre construction comportant un gène marqueur quelconque et contenant tous les éléments nécessaires en cis à son encapsidation, à sa réplication et à son intégration.

Une telle lignée LacZ$^+$ a été construite en introduisant la construction M-MuLVSV-LacZ de la fig. 1 dans la cellule NIH3T3 ATCC CCL92). Du virus sauvage MoMLV présent dans le surnageant d'une cellule chroniquement infectée a été mis en présence de la lignée NIH3T3 LacZ et 6 jours plus tard la présence de RVR-LacZ a été testée en transférant le surnageant de ces cultures sur des cellules 3T3NIH vierges. Comme indiqué dans le tableau 2, des virus sauvages ont été détectés avec une sensibilité mille fois meilleure que par le test F$^+$L$^-$ classiquement utilisé (FISCHINGER et al, Virology 59, 2317).

## TABLEAU 2

| Dilution du virus sauvage | Nombre de virus dRVR-LacZ après 3 jours | Test F$^+$L$^-$ |
|---|---|---|
| $10^{-7}$ | – | 100 |
| $10^{-8}$ | 80 | 10 |
| $10^{-9}$ | 14 | 1 |
| $10^{-10}$ | 5 | 0 |
| $10^{-11}$ | 2 | 0 |

Bien entendu, les conditions expérimentales qui précèdent n'ont aucun caractère limitif. Elles peuvent être aménagées en fonction de chaque type de rétrovirus et les conditions expérimentales recherchées, par exemple lorsqu'il s'agit d'apprécier l'effet d'un principe actif de médicament dont doit être étudie l'effet inhibiteur potentiel à l'égard du pouvoir infectieux du rétrovirus à l'égard des cellules étudiées.

Comme il va soi et comme il résulte d'ailleurs déjà de ce qui précède, l'invention ne se limite nullement à ceux de ses modes d'application et de réalisation qui ont été plus spécialement envisagés ; elle en embrasse au contraire toutes les variantes ; en particulier l'invention concerne encore un rétrovirus recombinant contenant au moins une séquence hétérologue supplémentaire codant pour une activité biologique distincte, également susceptible d'être exprimée dans des cellules transfectées avec le rétrovirus recombinant selon l'invention, en particulier dans des essais visant à étudier la capacité d'un agent extérieur déterminé à inhiber ou non cette activité biologique distincte.

**Revendications**

1. Rétrovirus recombinant défectif dérivé d'un rétrovirus sauvage ou natif, contenant les parties agissant en "cis" du virus sauvage et assurant normalement l'empaquetage du rétrovirus, sa réverse-transcription en une molécule ADN et son intégration dans le génome de la cellule hôte, ainsi que le site de polyadénylation de cet ARN, ce rétrovirus recombinant étant plus particulièrement caractérisé par la délétioon d'au moins une partie sinon de la totalité des sequences codant pour les protéines virales du virus sauvage ou natif, la partie délétée étant suffisamment importante pour que soit interdite la réplication du rétrovirus défectif qui en résulte dans la cellule-hôte, et par la substitution à la partie délétée d'une séquence génique contenant elle-même une séquence codant pour une protéine choisie parmi celles qui peuvent agir en tant que marqueurs de cellules-hôtes infectées, lorsque celles-ci sont infectées par le rétrovirus recombinant défectif ainsi constitué.

2. Rétrovirus recombinant défectif selon la revendication 1, caractérisé en ce que la séquence codant pour la protéine-marqueur est un promoteur distinct du LTR5′ du rétrovirus sauvage.

3. Rétrovirus recombinant selon la revendication 1, caractérisé en ce que le promoteur est celui de la région LTR5′ du rétrovirus.

4. Rétrovirus recombinant défectif qui comprend successivement :
- la région LTR5′ du rétrovirus sauvage correspondant, suivie de la région d'empaquetage ;
- le site d'initiation de l'ARN correspondant aux protéines virales normalement codées par les gènes gag, pol et env ;
- la séquence génique susdéfinie, concernant la séquence codant pour le marqueur, en lieu et place de tout ou partie de la région rétrovirale comportant ces gènes gag, pol et env, cette séquence génique étant placée sous le contrôle d'un promoteur approprié, le cas échéant de la région LTR5′ elle-même ;
- la région LTR3′, qui coïncide avec le site de polyadénylation de l'ARN.

5. Rétrovirus défectif recombinant caractérisé en ce que la séquence codant pour le marqueur code pour le bêta-galactosidase.

6. Lignée de cellules infectables par un rétrovirus sauvage ou natif déterminé, caractérisée en ce qu'elles contiennent dans leur patrimoine génétique un pro virus défectif recombinant dérivé du rétrovirus défectif recombinant, selon les revendications 1 à 5.

7. Procédé pour la détection du pouvoir infectieux d'un rétrovirus "sauvage" déterminé à l'égard de tout ou partie d'une culture cellulaire est donc caractérisé :
- par la mise en contact de cette culture cellulaire, avec un rétrovirus recombinant défectif tel que défini ci-dessus dans des conditions éventuellement susceptibles d'autoriser cette infection, ce rétrovirus recombinant défectif ayant en commun avec le rétrovirus sauvage les parties agissant en "cis" du virus sauvage (autrement dit les parties directement associées au rétrovirus recombinant) et assurant normalement la transcription virale, l'empaquetage du rétrovirus dans le génome de la cellule hôte, ainsi que le site de polyadénylation de cet ARN, et
- par la visualisation, le cas échéant à l'aide d'un révélateur approprié, de celles des cellules dans lesquelles le marqueur est exprimé.

8. Nécessaire ou kit pour la mise en oeuvre selon la revendication 6, réunissant :
- des rétrovirus recombinant selon l'une quelconque des revendications 1 à 5,
- le révélateur, notamment un substrat de l'enzyme consistant en le produit d'expression du marqueur de ce rétrovirus recombinant,
- le cas échéant, les tampons et réactifs nécessaires à la réalisation de la susdite mise en contact de ce rétrovirus recombinant avec la culture cellulaire dont est recherchée la sensibilité à l'infection par le rétrovirus sauvage correspondant, et ce en présence ou non d'agents extérieurs, tels que principes actifs de médicaments susceptibles d'interférer avec la manifestation du pouvoir infectieux du rétrovirus vis-à-vis de ces cellules sensibles.

9. Procédé de détection d'une infection rétrovirale dans un échantillon contenant des cellules (tel qu'un prélèvement de sérum humain contenant des lymphocytes T4, s'agissant d'apprécier s'il est infecté par un virus HIV) ou du milieu, tel qu'un surnageant de culture de ces cellules, auparavant au contact desdites cellules, ce procédé étant caractérisé par les étapes que constituent :
- la mise en contact de cet échantillon avec les cellules contenant un rétrovirus ou prophage défectif susceptible d'être lui-même complémenté par l'apport extérieur en rétrovirus éventuellement contenu dans l'échantillon, et ce dans des conditions (notamment de température et de durée d'incubation appropriées) permettant l'infection des cellules contenant le rétrovirus ou provirus recombinant défectif par le rétrovirus éventuellement présent dans l'échantillon, la réplication et la production alors induite du rétrovirus défectif correspondant dans le milieu d'incubation obtenu,
- la mise en contact du milieu d'incubation précédent, de préférence le surnageant de ce milieu, avec des cellules non infectées (cellules saines), néanmoins sensibles à l'infection par le rétrovirus sauvage, et ce dans des conditions permettant l'infection éventuelle desdites cellules saines placées au contact de ce milieu d'incubation ou de ce surnageant, et
- la détection de l'infection éventuelle desdites cellules antérieurement saines, par révélation de l'expression du marqueur incorporé au rétrovirus recombinant défectif, alors éventuellement présent dans ces dernières cellules, à l'aide d'un révélateur approprié.

10. Nécessaire ou kit pour la mise en oeuvre du procédé selon la revendication 8, caractérisé en ce qu'il comporte :
- des cellules sensibles modifiées contenant le rétrovirus recombinant défectif, selon l'une quelconque des revendications 1 à 5,
- des cellules semblables mais "saines" en ce qu'elles ne sont infectées ni par le rétrovirus recombinant défectif, ni par le rétrovirus sauvage,
- éventuellement les tampons et réactifs nécessaires à la réalisation, d'une part, d'une mise en contact des cellules sensibles mofiées avec l'échantillon biologique à étudier et l'incubation du mélange de réaction dans des conditions permettant l'infection éventuelle des cellules sensibles modifiées par l'enchantillon étudié et, d'autre part, d'une mise en contact des cellules saines avec les rétrovirus recombinants défectifs éventuellement libérés dans le milieu d'incubation des cellules sensibles modifiées infectées, après leur mise précédente en contact avec un prélèvement infecté par le rétrovirus à détecter,

- le cas échéant des milieux de conservation ou de culture pour lesdites cellules saines et modifiées, et
- des moyens de révélation, notamment un substrat de l'enzyme susceptible d'être exprimée par le marqueur incorporé au rétrovirus ou provirus recombinant défectif susdit.

11. Procédé pour la production du rétrovirus recombinant défectif, selon l'une quelconque des revendications 1 à 5.

- d'une lignée cellulaire auxiliaire contenant, incorporée à son propre ADN génomique, une séquence nucléique (ci-après dénommée "séquence de complémentation") capable de suppléer à l'absence dans le vecteur recombinant défectif des séquences, codant pour les protéines virales autorisant sa propagation dans les cellules qu'il est susceptible d'infecter. En particulier la séquence de complémentation contient les parties essentielles des gènes gag, pol et env du rétrovirus sauvage correspondant, assurant un apport "en trans" (autrement dit un apport extérieur) des séquences de nucléotides codant pour les protéines virales permettant la réplication, dans les cellules de cette lignée auxiliaire, d'un virus recombinant défectif contenant dans son génome les autres séquences rétrovirables du vecteur sauvage ;
- la culture de la lignée cellulaire auxiliaire ainsi transfectée dans des conditions permettant le réplication de ce rétrovirus recombinant in situ, grâce à l'apport par complémentation "en trans" des protéines virales codées par la susdite "séquence de complémentation" ;
- la récupération du virus recombinant défectif produit dans le surnageant de culture de ladite lignée celluliare auxiliaire.

12. Procédé selon la revendication 10 caractérisé en ce que la séquence de complémentation contient plus particulièrement la partie de l'ADN génomique du rétrovirus sauvage correspondant, codant notamment pour les protéines virales, à savoir les gènes gag, pol et env du rétrovirus sauvage correspondant, dont le vecteur recombinant défectif est lui-même dépourvu.

13. Nécessaire ou kit permettant la production "à la demande" du rétrovirus recombinant conforme à l'invention, les éléments essentiels de ces kits consistant :

- en un vecteur recombinant contenant le génome du rétrovirus conforme à l'invention ;
- en un hôte cellulaire auxiliaire susceptible d'être transfecté dans les conditions qui ont été décrites, pour produire le rétrovirus recombinant lui-même ; auxquels s'ajoutent éventuellement
- des tampons et réactifs nécessaires à la réalisation de la susdite transfection ;
- et, le cas échéant, un hôte cellulaire compétent, notamment bactérien, permettant une production amplifiée du susdit vecteur recombinant.

FIG.1

FIG.2

0273782

FIG. 3

FIG. 4

FIG.5A

SVnlsLacZ

LTR HIV-1    SphI        BamHI   LTR HIV-1    tat –
             989         8032

FIG.5B

SVnlsLacZ

LTR HIV-1    SphI        EcoRI   LTR HIV-1    tat +
             989         5289

RVsauvage

ou                                           α RRVLacZ    A tester sur
                                                          NIH 3T3 CEM
                                             RV           vierges

NIH 3T3      NIH 3T3MMuLVLacZ
(ATCC CC L92)  (CEM SW480 Fig 5A ou B)

             Test de détection du
                virus sauvage

FIG.6A       FIG.6B          FIG.6C       FIG.6D

| | Office européen des brevets | RAPPORT DE RECHERCHE EUROPEENNE | Numero de la demande |
|---|---|---|---|

EP 87 40 2336

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | EP-A-0 155 198  (INSERM)<br>* En entier, notamment figure 6 *<br>--- | 1-8,11, 12 | C 12 N  15/00<br>C 12 N   7/00<br>C 12 Q   1/70 |
| X | PROCEEDINGS OF THE NATIONAL ACADEMY OF SCIENCES USA, vol. 81, novembre 1984, pages 7137-7140; J.L.R. RUBENSTEIN et al.: "Construction of a retrovirus capable of transducing and expressing genes in multipotential embryonic cells"<br>* En entier *<br>--- | 1-8,11, 12 | |
| X,D | EP-A-0 178 220  (INSTITUT PASTEUR & CNRS)<br>* En entier *<br>--- | 1-4,6-8 ,11,12 | |
| X | EP-A-0 178 996  (INRA)<br>* Exemples 12,14 *<br>--- | 1-4,6-8 ,11,12 | |
| X | WO-A-8 600 922  (THE SALK INSTITUTE FOR BIOLOGICAL STUDIES)<br>* Exemple 1 *<br>--- | 1-4,6-8 ,11,12 | DOMAINES TECHNIQUES RECHERCHES (Int. Cl.4) |
| X | M. INOUYE: "Experimental manipulation of gene expression", 1983, chapitre 8, pages 155-173, Academic Press, New York, US; R.C. MULLIGAN: "Construction of highly transmissible mammalian cloning vehicles derived from murine retroviruses"<br>* Pages 159-164 *<br>----- | 1-4,6-8 ,11,12 | C 12 N<br>C 12 P<br>C 12 Q |

Le présent rapport a été établi pour toutes les revendications

| Lieu de la recherche | Date d'achèvement de la recherche | Examinateur |
|---|---|---|
| LA HAYE | 21-08-1988 | CUPIDO M. |